# EUROPEAN PATENT SPECIFICATION

(11) **EP 0 782 418 B1**
(45) Date of publication and mention of the grant of the patent: **22.12.1999**
(21) Application number: 95930745.5
(22) Date of filing: 14.09.1995
(51) Int. Cl.: A61F 2/32, A61F 2/34, A61F 2/36

(54) **ENDOPROSTHESIS**
ENDOPROTHESE
ENDOPROTHESE

(30) Priority: 16.09.1994 PL 30506094
(43) Date of publication of application: 09.07.1997
(73) Proprietor: Rogala, Piotr, 60-615 Poznan (PL)
(72) Inventor: Rogala, Piotr, 60-615 Poznan (PL)
(74) Representative: Grünecker, Kinkeldey, Stockmair & Schwanhäusser Anwaltssozietät
(86) International application number: PCT/PL95/00020
(87) International publication number: WO 96/08214

(56) References cited:
- EP-A- 0 009 148
- EP-A- 0 013 863
- FR-A- 2 519 545
- FR-A- 2 598 908
- FR-A- 2 686 503
- GB-A- 2 150 441
- US-A- 2 910 978
- US-A- 3 840 904

## Description

The subject of this invention is a bone endoprosthesis for implantation without using surgical cement.

In the known implantation methods, the connection between the endoprosthesis and the bone takes place by the growing into pores of proper dimensions, into indentations and other irregularities on the surface of the endoprosthesis, fixed on the spot where it is mounted with the help of surgical cement, or mechanically. In order to repair losses of long bones' shanks, among others, endoprostheses are used which are joined with the bone by wedging into the marrow cavity, using surgical cement, like for instance polymethacryl methyl, with the help of roughly rolled threads. This joining method is possible with metallic endoprostheses, which, however, have the imperfection that there is a very small surface for the ingrowth of the spongy bone.

Besides, the joining of the endoprosthesis to the bone by surgical cement has some defects. The main defect is that the material weakens as it ages and may burst. Consequently, irritation from the failing cement causes a separation from the bone and this, in turn, may loosen of the endoprosthesis and causes atrophy of the bone.

DE3443109A1 describes a prosthesis in which the prosthetic implants have more secure fastenings by applying conic irregularities which stick out of the terminal surface of the endoprosthesis and which are directed against the joining surface of the bone. The conic irregularities are pressed into the spongy bone and cause an increase of the contact surface which is favorable to the fixation, thanks to bone ingrowth.

An imperfection of this solution is the fact that there is no possibility to optimally increase the contact surface of the endoprosthesis with the bone because the conic irregularities are spaced apart on a plane surface. However, the plane contact surface, because of its shape, renders impossible ingrowth of spongy bone in a shape similar to the natural form, which would assure a good absorption of the joint's forces.

The aim of the invention is the elimination of these imperfections by creating an endoprosthesis, thanks to which there is a simple and durable union with the bone tissue, with the cortex, as well as with the spongy tissue, ensuring at the same time the possibility to rebuild the regenerated bone in a form similar to the natural and original one.

This aim has been achieved with the endoprosthesis according to this invention, which is implanted with the help of needles in the terminal surface of the spongy bone, at first by a central needle and then by outer needles in such a way that the shape of the terminal surface enables the ingrowth of osteoblasts to reshape the bone in an external form similar to the natural one.

An endoprosthesis according to the preamble of claim 1 is known from document FR-A-2 519 545. The present invention is recited in claim 1.

The implantation of the endoprosthesis of the invention is realized by removing the defective tissue from the bones which are part of the joint, by giving them a form similar to the configuration of the peaks of the multilateral needles which are introduced into the spongy bones, by and by, and by maintaining the endoprosthesis aligned with a central axis of the operated glenoid cavity of the bone as well as the operated bone head. Along the axis of the bone's head, the central needle of the endoprosthesis' head is introduced simultaneously into the spongy bone in order to assure a contact of its resistance surface with the periosteum, with the cortex and with the adjacent spongy structured lamella, in which are introduced needles lying in a greater distance than the plane of the resistance surface. The multilateral needles of the glenoid cavity and of the head are successively and symmetrically introduced into the spongy bone up to the resistance of the resistance edge of the glenoid cavity against the bone, as well as the resistance surface of the head up to the resistance with the essential cortex and the adjacent lamella, which has a spongy structure. The distance between the remaining needles which have not been introduced into the spongy bone and the terminal surface is filled up in "biological silence" by osteoblasts.

The glenoid cavity of the endoprosthesis of the invention is implanted in such a manner that the resistance edge lies on the bone perpendicularly to the central axis of the acetabulum of the bone. The resistance edge is placed symmetrically about the axis. The needles which are near the spherical belt on the terminal surface of the endoprosthesis are, simultaneously, partially introduced into the spongy structure of the bone. The remaining surface between the multilateral needles which are not introduced into the spongy bone down to the terminal surface is filled by osteoblasts in "biological silence".

The endoprosthesis according to the invention is composed of an acetabulum and a head which, on their terminal surfaces, have multilateral needles with parallel axes and different lengths, the whole surface of which makes the multiplicity of the joint's surface, whilst the edges of the bases of adjacent needles adjoin each other, and their axes are adequately perpendicular to the surface in which the resisting edge of the acetabulum as well as the resisting surface of the head is located.

An embodiment of an endoprosthesis according to the invention is described with the help of the enclosed drawings where:

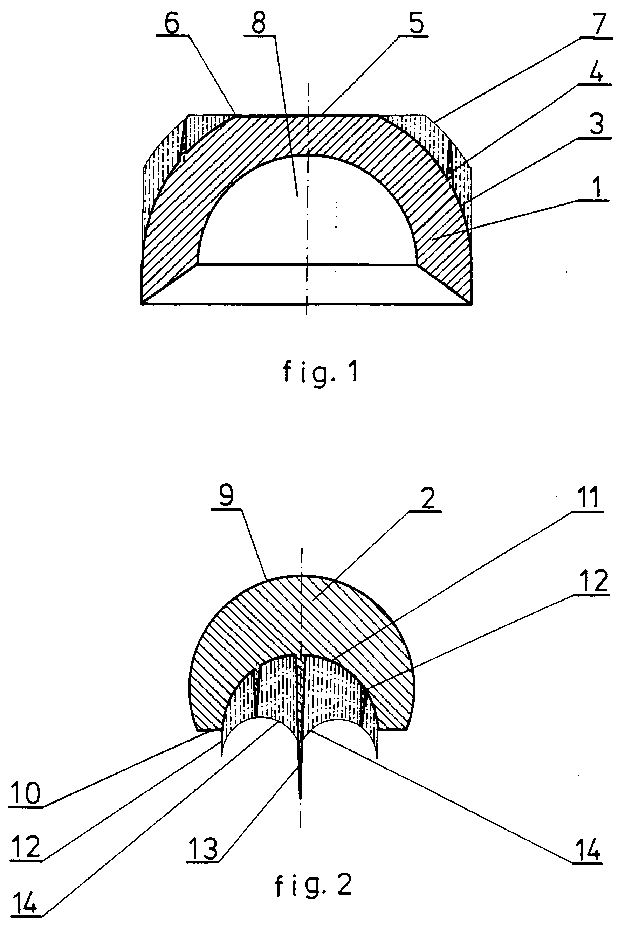

figure 1 schematically illustrates the acetabulum of the endoprosthesis in cross-section; and
figure 2 schematically illustrates the head of the endoprosthesis in cross-section.

The endoprosthesis is composed of an acetabulum 1 and a head 2. The acetabulum 1 has an exterior spherical terminal surface 3 which is equipped with multilateral needles 4 with axes parallel to each other. Each needle is parallel to the central axis of the acetabulum. The circular surface 5 which is determined by the resistance edge 6 is parallel to the central axis of the acetabulum. The multilateral needles 4 have common base edges (not shown in the drawing) with adjacent needles 4 and differing lengths. The length of a multilateral needle 4 as measured from the base on the terminal surface 3 is determined jointly by the central and the terminal surface 3, by the theoretical spherical surface 7 which crosses the peaks of a part of the multilateral needles 4 as well as by the plane in which the circular surface 5 is located.

The outermost multilateral needles 4 do not extend beyond the tangent to the terminal surface 3 which is parallel to the axis of the acetabulum. Those needles which lie close to the resistance edge 6 have the least length.

The acetabulum 1 comprises a pan 8 to place the head 2 which constitutes a part of the sphere with the external surface 9, and has an annular resistance surface 10 and a spherical terminal surface 11. On the spherical terminal surface 11 there are multilateral needles 12 and a central multilateral needle 13 with mutually parallel axes. The multilateral central needle is coincident with the axis of the head 2.

The multilateral needles 12 have common base edges (not shown in the drawings) with neighboring multilateral needles 12 and differing lengths.

The exterior multilateral needles 12 which are situated close to the resistance surface 10 have an advantageous ratio of one-half of the base diagonal to their height of 1:5. The peak of the multilateral central needle 13 lies on the circumference of the sphere which creates the external surface 9 of the head. The remaining lengths of the multilateral needles 12 are determined by the distance between their bases on the terminal surface 11 and their peaks which lie on the theoretical surface of the bowls 14 with a radius equal to half the distance between the axis of the multilateral needle 12 and the axis of the central multilateral needle 13, carried out from the point between the axes on the straight line which connects the peaks of the opposite extreme multilateral needles 12.

The surfaces of the multilateral needles 12 and the surfaces of the central multilateral needle 13 have a total surface which is seven times bigger than the surface 9. This reflects approximately the internal surface of the essence of the bone bark on which the head is implanted. Simultaneously, the different lengths of the symmetrically placed multilateral needles 12 enable a steady and successive introduction into the spongy bone.

The resistance edge 6 as well as the resistance surface 10 properly bear on the bone of the pelvis acetabulum and on the bark edge of the bone tight femoral neck. The needles' composition considerably increases the adhesion when bearing on the spongy bone and this causes a more effective absorption of the forces. Moreover, it increases the fixation power in the bone tissue, thus preventing the endoprosthesis from spraining and loosening.

The endoprosthesis is produced in various dimensions properly fixed according to the gradation of biological sizes, and from biologically non-toxic materials, and is possessing the proper mechanical properties.

The acetabulum as well as the head, among others, can be produced entirely out of plastic with a varying density gradation so that its resistance power is a function of the varying elasticity of the plastic, decreasing in due measure from the surface in both parts of the endoprosthesis. Due to the mass, in an almost natural way, suppression of the forces which occur in the joint is obtained.

One of the advantages of the endoprosthesis is the proper arrangement of power between the bone and the endoprosthesis. Moreover, the operation connected with the implantation is simplified and can be performed with sick people in a considerably wider age range.

Another advantage is the longer durability of the endoprosthesis and, if necessary, it is easy to replace, as the endoprosthesis in its shape is always very close to the biological model. At the same time, the endoprosthesis facilitates its application with patients with a big osteoporosis and enables the implantation without using surgical cement.

Also it should be emphasized that the implantation of the endoprosthesis according to this invention causes less injuries of the bone tissues.

The endoprosthesis of the invention is a hip prosthesis which can be applied with appropriate in operations on humans and animals.

The subject of this invention can be applied also in all other cases of similar biomechanical properties.

## Claims

1. Endoprosthesis for a joint comprising an acetabulum (1) and a head (2), both having multilateral needles (4, 12, 13) on their terminal surfaces (3, 11) which are directed against the bone, wherein the terminal surface (3) of the acetabulum (1) is a convex annular surface, wherein the multilateral needles (4, 12, 13) of both the acetabulum (1) and the head (2) have mutually parallel axes and are situated symmetrically about the respective center axes of the acetabulum (1) and the head (2); and wherein the multilateral needles of the head (2) include a central multilateral needle (13), **characterized in that** the terminal surface (11) of the head (2) is concave and surrounded by an annular resistance surface (10) which contacts the spherical external surface (9) of the head (2) for contacting the acetabulum (1), each of said multilateral needles (4, 12) of the acetabulum (1) and the head (2) contacts its immediately adjacent multilateral needles (4, 12), the multilateral needles (4, 12, 13) of both the acetabulum (1) and the head (2) have differing lengths whereby the multilateral needles (4) close to the resistance edge (6) of the acetabulum (1) and the multilateral needles (12) close to the resistance surface (10) of the head (2) are the shortest, and the total surface of all the multilateral needles (12, 13) of the head (2) is seven times larger than the spherical external surface (9) of the head (2).

2. Endoprosthesis according to Claim 2 **characterized in that** the resistance edge (6) of the acetabulum (1) as well as the resistance surface (10) of the head (2) are situated in parallel planes perpendicular to the respective central axes of the acetabulum (1) and the head (2), and in that the central needle (13) of the head (2) is coincident with the central axis of the head (2) and is longer than the other multilateral needles (12) of the head (2).

3. Endoprosthesis according on one of Claims 1 and 2 **characterized in that** the multilateral needles (12) of the head (2) which are situated close to the resistance surface (10) have a length which is five times larger than half of their base diagonal.

4. Endoprosthesis according to one of the above Claims **characterized in that** the peak of the central multilateral needle (13) of the head (2) lies on the circumference of the sphere which creates the spherical external surface (9) of the head (2).

5. Endoprosthesis according to one of the above Claims **characterized in that** the acetabulum (1) forms a pan (8) which receives the head (2).

6. Endoprosthesis according to one of the above Claims **characterized in that** the resistance edge (6) of the acetabulum (1) delimits a planar circular surface (5) which is perpendicular to the central axis of the acetabulum (1).

7. Endoprosthesis according to one of the above Claims **characterized in that** the multilateral needles (12) of the head (2) have common base edges with immediately adjacent multilateral needles (12).

8. Endoprosthesis according to one of the above Claims **characterized in that** the multilateral needles (4) of the acetabulum (1) have common base edges with immediately adjacent multilateral needles (4).

## Patentansprüche

1. Endoprothese für ein Gelenk, umfassend ein Azetabulum (1) und einen Kopf (2), die beide multilaterale Nadeln (4, 12, 13) an ihren Endflächen (3, 11) aufweisen, welche gegen den Knochen gerichtet sind, wobei die Endfläche (3) des Azetabulums (1) eine konvexe, ringförmige Fläche ist, wobei die multilateralen Nadeln (4, 12, 13) sowohl des Acetabulums (1) als auch des Kopfes (2) zueinander parallele Achsen aufweisen und symmetrisch um die jeweiligen Mittelachsen des Azetabulums (1) und des Kopfes (2) angeordnet sind, und wobei die multilateralen Nadeln des Kopfes (2) eine zentrale multilaterale Nadel (13) aufweisen, **dadurch gekennzeichnet**, daß die Endfläche (11) des Kopfes (2) konkav und von einer ringförmigen Widerstandsfläche (10) umgeben ist, die die kugelförmige äußere Oberfläche (9) des Kopfes (2) zur Berührung des Azetabulums (1) berührt, daß jede der multilateralen Nadeln (4, 12) des Azetabulums (1) und des Kopfes (2) ihre unmittelbar benachbarten multilateralen Nadeln (4, 12) berührt, und daß die multilateralen Nadeln (4, 12, 13) sowohl des Azetabulums (1) als auch des Kopfes (2) unterschiedliche Längen aufweisen, wobei die multilateralen Nadeln (4) nahe der Widerstandskante (6) des Azetabulums (1) und die multilateralen Nadeln (12) nahe der Widerstandsfläche (10) des Kopfes (2) am kürzesten sind, und die Gesamtfläche aller multilateralen Nadeln (12, 13) des Kopfes (2) siebenmal größer als die kugelförmige äußere Fläche (9) des Kopfes (2) ist.

2. Endoprothese nach Anspruch 2, **dadurch gekennzeichnet**, daß sowohl die Widerstandskante (6) des Azetabulums (1) als auch die Widerstandsfläche (10) des Kopfes (2) in parallelen Ebenen senkrecht zur jeweiligen Mittelachse des Azetabulums (1) und des Kopfes (2) gelegen sind, und daß die zentrale Nadel (13) des Kopfes (2) mit der Mittelachse des Kopfes (2) zusammenfällt und länger ist als die anderen multilateralen Nadeln (12) des Kopfes (2).

3. Endoprothese nach einem der Ansprüche 1 und 2, **dadurch gekennzeichnet**, daß die multilateralen Nadeln (12) des Kopfes (2), die nahe der Widerstandsfläche (10) gelegen sind, eine Länge aufweisen, die fünfmal größer ist als die Hälfte ihrer Grundflächendiagonale.

4. Endoprothese nach einem der obigen Ansprüche, **dadurch gekennzeichnet**, daß die Spitze der zentralen multilateralen Nadel (13) des Kopfes (2) auf dem Umfang der Kugel liegt, die die kugelförmige äußere Fläche (9) des Kopfes (2) bildet.

5. Endoprothese nach einem der obengenannten Ansprüche, **dadurch gekennzeichnet**, daß das Azetabulum (1) eine Pfanne (8) ausbildet, die den Kopf (2) aufnimmt.

6. Endoprothese nach einem der obigen Ansprüche, **dadurch gekennzeichnet**, daß die Widerstandskante (6) des Azetabulums (1) eine ebene kreisförmige Fläche (5) begrenzt, die senkrecht zur Mittelachse des Azetabulums (1) verläuft.

7. Endoprothese nach einem der obengenannten Ansprüche, **dadurch gekennzeichnet**, daß die multilateralen Nadeln (12) des Kopfes (2) gemeinsame Grundflächenkanten mit unmittelbar benachbarten multilateralen Nadeln (12) aufweisen.

8. Endoprothese nach einem der obengenannten Ansprüche, **dadurch gekennzeichnet**, daß die multilateralen Nadeln (4) des Acetabulums (1) gemeinsame Grundflächenkanten mit unmittelbar benachbarten multilateralen Nadeln (4) aufweisen.

## Revendications

1. Endoprothèse pour une articulation, comportant une acétabule (1) et une tête (2) pourvues toutes les deux sur leurs surfaces terminales (3, 11) d'aiguilles multilatérales (4, 12, 13) qui sont dirigées vers l'os, étant précisé que la surface terminale (3) de l'acétabule (1) est une surface annulaire convexe, que les aiguilles multilatérales (4, 12, 13) de l'acétabule (1) et de la tête (2) ont des axes parallèles et sont situées symétriquement par rapport aux axes centraux respectifs de l'acétabule (1) et de la tête (2), et que les aiguilles multilatérales de la tête (2) comportent une aiguille multilatérale centrale (13), **caractérisée** en ce que la surface terminale (11) de la tête (2) est concave et est entourée par une surface de résistance annulaire (10) qui est en contact avec la surface extérieure sphérique (9) de la tête (2) pour être en contact avec l'acétabule (1), chacune des aiguilles multilatérales (4, 12) de l'acétabule (1) et de la tête (2) est en contact avec les aiguilles multilatérales (4, 12) directement voisines, les aiguilles multilatérales (4, 12, 13) de l'acétabule (1) et de la tête (2) ont des longueurs différentes, les aiguilles multilatérales (4) proches du bord de résistance (6) de l'acétabule (1) et les aiguilles multilatérales (12) proches de la surface de résistance (10) de la tête (2) étant les plus courtes, et la surface totale de toutes les aiguilles multilatérales (12, 13) de la tête (2) est sept fois plus grande que la surface extérieure sphérique (9) de la tête (2).

2. Endoprothèse selon la revendication 1, **caractérisée** en ce que le bord de résistance (6) de l'acétabule (1) et la surface de résistance (10) de la tête (2) sont situés dans des plans parallèles qui sont perpendiculaires aux axes centraux respectifs de l'acétabule (1) et de la tête (2), et en ce que l'aiguille centrale (13) de la tête (2) coïncide avec l'axe central de celle-ci et est plus longue que les autres aiguilles multilatérales (12) de la tête (2).

3. Endoprothèse selon l'une des revendications 1 et 2, **caractérisée** en ce que les aiguilles multilatérales (12) de la tête (2) qui sont situées près de la surface de résistance (10) ont une longueur qui est cinq fois plus grande que la moitié de leur diagonale de le base.

4. Endoprothèse selon l'une des revendications précédentes, **caractérisée** en ce que la pointe de l'aiguille multilatérale centrale (13) de la tête (2) se trouve sur la circonférence de la sphère qui définit la surface extérieure sphérique (9) de la tête (2).

5. Endoprothèse selon l'une des revendications précédentes, **caractérisée** en ce que l'acétabule (1) forme une cavité (8) qui reçoit la tête (2).

6. Endoprothèse selon l'une des revendications précédentes, **caractérisée** en ce que le bord de résistance (6) de l'acétabule (1) délimite une surface circulaire plane (5) qui est perpendiculaire à l'axe central de ladite acétabule (1).

7. Endoprothèse selon l'une des revendications précédentes, **caractérisée** en ce que les aiguilles multilatérales (12) de la tête (2) ont des bords de base communs avec les aiguilles multilatérales (12) directement voisines.

8. Endoprothèse selon l'une des revendications précédentes, **caractérisée** en ce que les aiguilles multilatérales (4) de l'acétabule (1) ont des bords de base communs avec les aiguilles multilatérales (4) directement voisines.
